# EUROPÄISCHE PATENTSCHRIFT

(11) **EP 1 097 343 B1**
(45) Veröffentlichungstag und Bekanntmachung des Hinweises auf die Patenterteilung: **18.09.2002**
(21) Anmeldenummer: 99931102.0
(22) Anmeldetag: 18.06.1999
(51) Int. Cl.: F25B 13/00

(54) **ANLAGE ZUR REGELUNG DES BETRIEBS EINER EINRICHTUNG FÜR DIE LAGERUNG UND VERTEILUNG VON KOHLENDIOXID**
FACILITY FOR REGULATING THE OPERATION OF A DEVICE FOR STORING AND DISTRIBUTING CARBON DIOXIDE
INSTALLATION PERMETTANT DE REGULER LE FONCTIONNEMENT D'UN DISPOSITIF DE STOCKAGE ET DE DISTRIBUTION DE DIOXYDE DE CARBONE

(30) Priorität: 10.07.1998 FR 9809068
(43) Veröffentlichungstag der Anmeldung: 09.05.2001
(73) Patentinhaber: Messer France S.A., 93206 Saint Denis Cedex (FR)
(72) Erfinder: MATHEOUD, Patrick, F-92390 Villeneuve La Garenne (FR); BEAUGE, Claude, F-95610 Eragny-sur-Oise (FR); GILBERT, Ghislain, F-60800 Ormoy Villers (FR); LESNEK, Stéphane, F-95130 Franconville (FR)
(74) Vertreter: Berdux, Klaus, Dipl.-Ing.
(86) Internationale Anmeldenummer: EP9904233
(87) Internationale Veröffentlichungsnummer: WO00003187

(56) Entgegenhaltungen:
- WO-A-91/10477
- WO-A-97/31230
- US-A- 3 754 407
- US-A- 4 897 226
- US-A- 5 255 523

## Beschreibung

Gegenstand dieser Erfindung ist eine Anlage zur Regelung des Betriebs einer Einrichtung für die Lagerung und Verteilung von Kohlendioxid gemäß dem Oberbegriff des Anspruch 1. Eine Solche Anlage ist aus Dokument WO-A-9 110 477 schon bekannt.

Zwischen den optimalen Verteilungsbedingungen und den Lagerungsbedingungen besteht häufig eine Diskrepanz.

Deshalb müssen derartige Einrichtungen mit technischen Mitteln ausgestattet werden, die es ermöglichen, den spezifischen Einsatzbedingungen solcher Einrichtungen Rechnung zu tragen.

Bekanntlich wird eine derartige Einrichtung mit einem Aggregat zur Kälteerzeugung ausgerüstet, so daß die Gasphase innerhalb eines Behälters gekühlt und ein zu hoher Druckanstieg vermieden werden kann. Es gibt jedoch keine Möglichkeit, den Druck, wenn dieser während der Anwendung unter eine zulässige Schwelle absinkt, erforderlichenfalls wieder zu erhöhen.

Außerdem wird eine derartige Einrichtung bekanntlich mit einer oder mehreren Maschinen ausgerüstet, die dem flüssigen Kohlendioxid Kältekalorien zuführen, damit dieses unterkühlt und seine Anwendung in flüssiger Form ermöglicht wird. Auch hier handelt es sich jedoch um eine spezifische Ausrüstung, die für andere spezifische Anwendungssituationen einer Einrichtung zur Lagerung und Verteilung von flüssigem Kohlendioxid nicht geeignet ist.

Und bekanntlich muß auch einer Forderung zur Lieferung von flüssigem Kohlendioxid, das jedoch unmittelbar vor einer mit gasförmigem Kohlendioxid zu speisenden Anwendungsanlage verdampft werden muß, entsprochen werden. Dazu wird bekanntlich ein elektrischer Erhitzer, der als spezifische Ausrüstung über seine eigene Energieversorgung verfügen muß, oder ein Lufterhitzer verwendet, dessen Hauptnachteil darin besteht, daß er regelmäßig abgetaut werden muß. Eine solche Anlage ist aus Dokument WO-A-9 110 477 schon bekannt.

Mit anderen Worten, um allen möglichen Anwendungserfordernissen einer Einrichtung zur Lagerung und Verteilung von flüssigem Kohlendioxid gerecht zu werden, ist eine verhältnismäßig umfangreiche Palette spezifischer Ausrüstungen erforderlich, die bei der Lagerung, der nachfragegerechten Verfügbarkeit, der Wartung und der Bereitstellung eigenständiger Energieversorgung Probleme verursachen, deren praktische Auswirkung darin besteht, daß die Kosten für Erwerb und Wartung einer Lagerungs- und Verteilungseinrichtung steigen.

Es zeigt sich also Bedarf an einem technischen Mittel, das mit jeder Einrichtung zur Lagerung und Verteilung von flüssigem Kohlendioxid mitgeliefert werden kann und eine Anpassung des Betriebs ermöglicht, so daß alle spezifischen Erfordernisse, die sich aus der speziellen Anwendung, für die die Einrichtung vorgesehen ist, ergeben, erfüllt werden können.

Eines der Ziele der Erfindung besteht darin, diesem Bedarf gerecht zu werden und eine Anlage zur Regelung des Betriebs anzubieten, die leicht an die Einrichtungen zur Lagerung und Verteilung von Kohlendioxid angepaßt werden kann, verhältnismäßig einfach zu installieren ist und die Verwendung technischer Mittel ermöglicht, durch die die Kosten für Investitionen, Wartung und Energie, die dem Anwender durch die technischen Mittel des Standes der Technik entstehen, wenn er die spezifischen Anwendungsbedingungen erfüllen will, gesenkt werden können.

Zur Erreichung der obengenannten Ziele ist die Anlage zur Regelung des Betriebs einer Einrichtung für die Lagerung und Verteilung von Kohlendioxid dadurch gekennzeichnet, daß sie folgendes umfaßt:
- eine reversible Kältemaschine,
- einen ersten Austauscherkreislauf mit einem Austauscher in dem Teil des Behälters, der von der Gasphase des Kohlendioxids eingenommen wird,
- einen zweiten Austauscherkreislauf mit einem Austauscher in der Ableitungsstrecke und
- zwei Steuerventile mit einander zugeordneten entgegengesetzten Steuerungen, die zwischen der Kältemaschine und dem ersten bzw. zweiten Kreislauf angeordnet sind.

Verschiedene weitere Merkmale ergeben sich aus der nachfolgenden Beschreibung, in der auf die beigefügten Zeichnungen Bezug genommen wird, die als nicht begrenzendes Beispiel eine Ausführungsform des Gegenstands der Erfindung zeigen.

Die Figuren 1, 1a und 1b sind schematische Darstellungen, die die erfindungsgemäße Anlage zur Betriebsregelung zeigen.

Die Figuren 2 bis 6 veranschaulichen bestimmte charakteristische Phasen der Betriebsregelung.

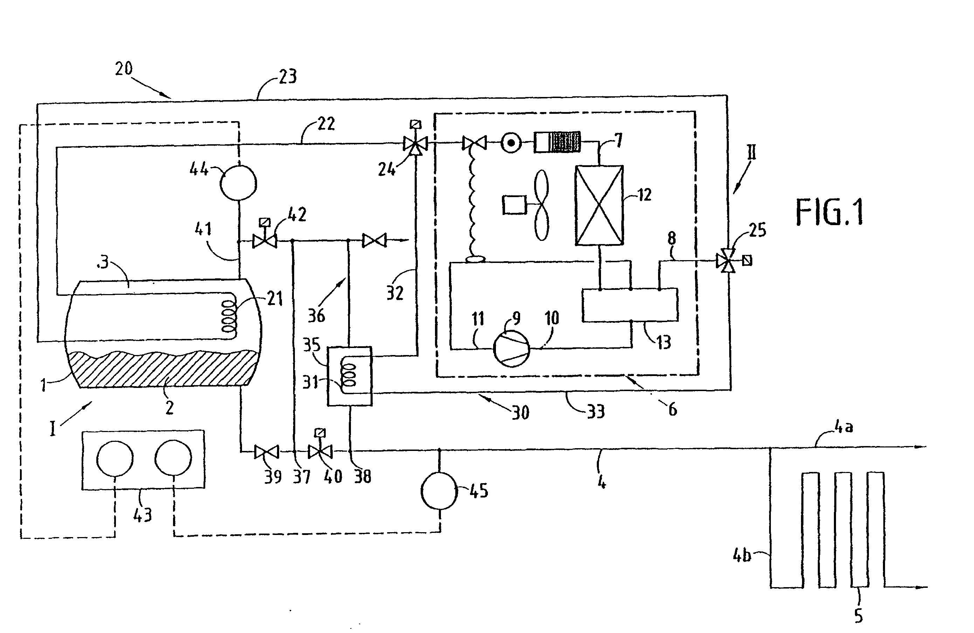

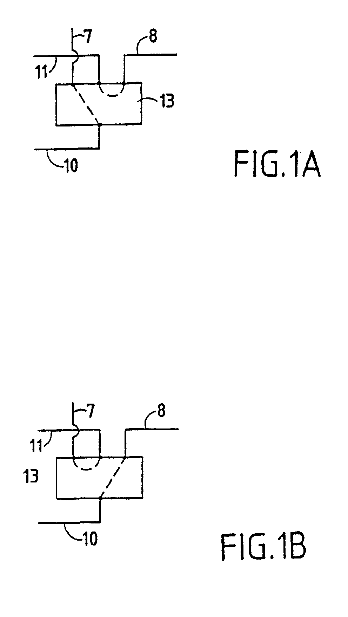

Figur 1 zeigt die erfindungsgemäße Betriebsregelungsanlage, die an eine Einrichtung I zur Lagerung und Verteilung von Kohlendioxid angepaßt ist. Eine derartige Einrichtung hat einen Behälter 1, der zum Teil mit einer Masse 2 flüssigen Kohlendioxids gefüllt ist, über der sich eine Decke 3 aus gasförmigem Kohlendioxid unter Druck befindet. Der Behälter 1 ist an eine Entnahmeleitung 4 angeschlossen, die zu einem Anwendungsgerät führt, das nicht direkt Gegenstand der Erfindung ist, dessen spezifischer Charakter jedoch entweder eine Versorgung mit flüssigem Kohlendioxid, wie schematisch durch die Abzweigung 4a dargestellt, oder eine Versorgung mit gasförmigem Kohlendioxid erforderlich macht, wie schematisch durch die Abzweigung 4b dargestellt, die dann vor dem nicht dargestellten Gerät einen Luftaustauscher 5 zur Verdampfung des Kohlendioxids hat.

Die Betriebsregelungsanlage, die insgesamt mit II bezeichnet ist, hat eine reversible Kältemaschine 6, die eine erste Strömungsleitung 7 sowie eine zweite Strömungsleitung 8 hat, zwischen denen ein Verdichter 9 mit einem Austrittsoder Druckstutzen 10 und einem Eintritts- oder Einlaßstutzen 11 angeordnet ist. Die Maschine 6 hat einen Austauscher 12 in der ersten Leitung 7, die ebenso wie die Leitung 8 an einen Umkehrblock 13 angeschlossen ist, an dem auch die Stutzen 10 und 11 des Verdichters enden.

Der Umkehrblock 13 ist so konzipiert, daß, wie in Figur 1a dargestellt, die Verbindung zwischen der Leitung 8 und dem Stutzen 11 und gleichzeitig zwischen dem Stutzen 10 und der Leitung 7 vor dem Austauscher 12 gewährleistet werden kann.

Außerdem ist der Umkehrblock 13 so konzipiert, daß, wie in Figur 1b dargestellt, die Verbindung zwischen der Leitung 8 und dem Stutzen 10 und gleichzeitig zwischen dem Stutzen 11 und der ersten Leitung 7 hergestellt werden kann.

Die Anlage II umfaßt darüber hinaus einen ersten Austauschkreislauf 20 mit einem Austauscher 21, der in der Decke 3 von Behälter 2 angeordnet ist. Der Austauscher 21 ist an zwei Leitungen 22 und 23 angeschlossen, die über Ventile 24 und 25 mit der Leitung 7 bzw. 8 von Maschine 6 verbunden sind.

Die Anlage verfügt zudem über einen zweiten Austauschkreislauf 30 mit einem Austauscher 31, dessen Leitungen 32 und 33 ebenfalls über die Ventile 24 und 25 an die Leitungen 7 und 8 angeschlossen sind. Aus diesem Grund handelt es sich bei den Ventilen 24 und 25 um Dreiwegeventile, die z.B. elektrisch oder pneumatisch gesteuert werden und deren Besonderheit, wie im weiteren zu sehen sein wird, in den einander zugeordneten entgegengesetzten Steuerungen besteht. Der Austauscher 31 befindet sich in einer Kammer 35, die in einer Umgehungsschleife 36 angeordnet ist, die über Anschlußstellen 37 und 38 an die Entnahmeleitung 4 angeschlossen ist. Die Anschlußstelle 37 liegt hinter einem Hahn 39 in der Entnahmeleitung 4 am Ausgang von Behälter 1. Die Entnahmeleitung 4 hat zwischen den Anschlußstellen 37 und 38 ein Abschalt-Regelungsventil 40.

Nach einer weiteren Anordnung der Erfindung ist die Umgehungsschleife 36 mit der Behälterdecke 3 über eine Abzweigung 41 mit einem Absperrhahn 42 verbunden.

Zur Anlage gehören vorzugsweise weiterhin Mittel zur automatischen Betriebsregelung 43, die insbesondere durch einen Drucksensor 44 in der Abzweigung 41 zwischen Behälter 1 und Hahn 42 und durch einen Temperatursensor 45 in der Leitung 4 hinter der Anschlußstelle 38 gesteuert werden.

Die oben beschriebene Betriebsregelungsanlage ermöglicht die Herstellung folgender Betriebs-bedingungen.

Figur 1 veranschaulicht eine Betriebssituation, in der die reversible Kältemaschine 6 abgeschaltet und der Hahn 42 geschlossen ist, während Hahn 39 und Ventil 40 geöffnet sind. Der Hahn 39, dessen Funktion darin besteht, für Wartungsarbeiten eine Abschaltung zu ermöglichen, ist normalerweise stets geöffnet.

Die Entnahmeleitung 4 gibt dann an den Anwender Kohlendioxid in der flüssigen Phase ab, das über die Abzweigung 4a oder über die Abzweigung 4b ein Anwendungsgerät versorgen kann.

Es kann vorkommen, daß sich der Austauscher 12 aufgrund einer früheren Betriebsart im Zustand einer Vereisung befindet, die für eine anschließende optimale Nutzung beseitigt werden muß.

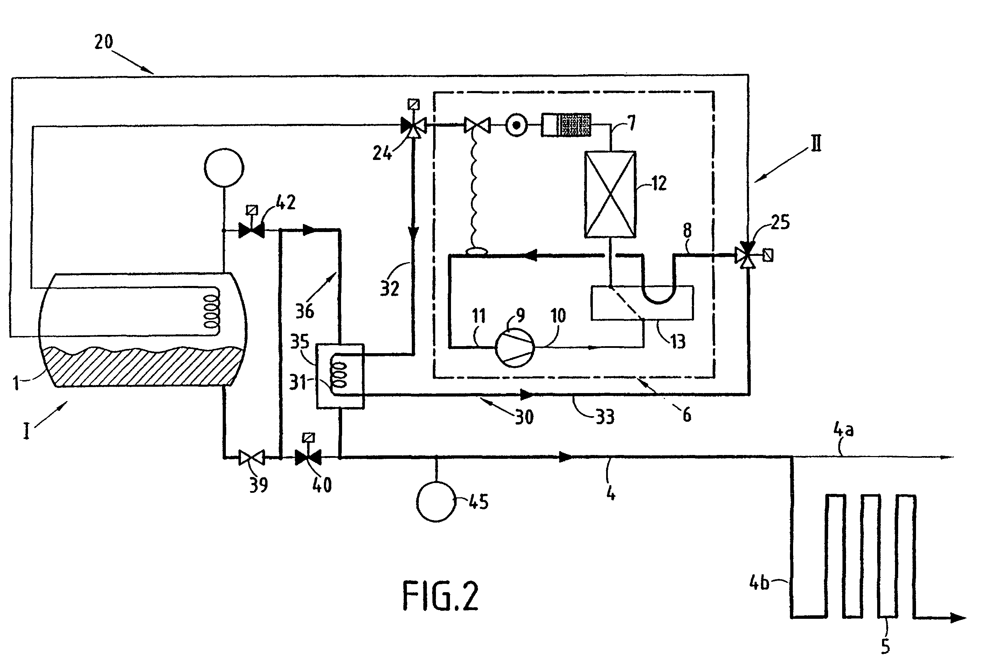

Figur 2 veranschaulicht die Inbetriebnahme der Anlage zur Lösung dieses Problems unter Berücksichtigung der vorangegangenen Betriebsart, d. h. der Entnahme von flüssigem Kohlendioxid.

Nach Figur 2 bleibt in der dargestellten Situation der Anlage das Ventil 42 geschlossen, das Ventil 39 geöffnet, während das Ventil 40 geschlossen wird. Gleichzeitig werden die Ventile 24 und 25 so gesteuert, daß die Maschine 6 mit dem Kreislauf 30 verbunden wird, und der Block 13 betätigt, damit der Stutzen 10 an den Austauscher 12 angeschlossen wird, während der Stutzen 11 an die Leitung 8 angeschlossen ist.

Das flüssige Kohlendioxid gelangt damit in die Schleife 36 und wird durch die Kammer 35 geführt. Durch die Inbetriebnahme der Maschine 6 wird die Leitung 7 mit einem komprimierten gasförmigen Fluid versorgt, das durch den Austauscher 12 strömt, in dem es kondensiert wird, wobei es diesen abtaut. Die in diesem Fluid gespeicherten Kältekalorien werden innerhalb des Austauschers 31, der in der Kammer 35 in das flüssige Kohlendioxid getaucht ist, wirkungslos freigesetzt.

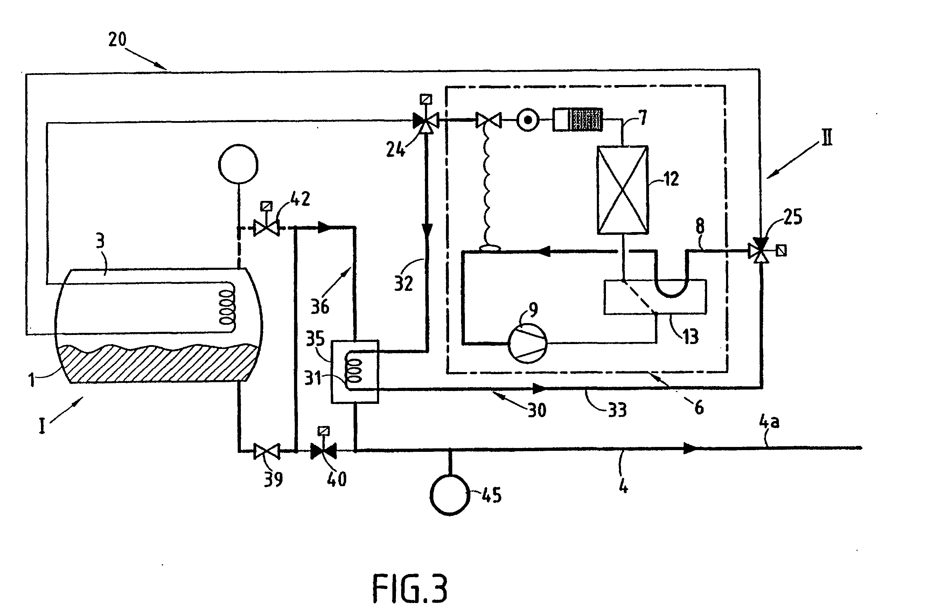

Diese Betriebsphase kann, wie in Figur 3 dargestellt, genutzt werden, um flüssiges Kohlendioxid, das z.B. ein Anwendungsgerät über die Abzweigung 4a versorgen soll, zu unterkühlen. In einem solchen Fall kann es auch vorteilhaft sein, die Öffnung des Ventils 42 so einzustellen, daß ein Teil des gasförmigen Kohlendioxids zu dem Zweck durchgelassen wird, die Temperatur des über die Leitung 4 strömenden Kohlendioxids zu regeln. Die Regelung der Öffnung von Ventil 42 kann in Abhängigkeit vom Temperatursensor 45 erfolgen.

Diese Betriebsphase kann auch für eine Verteilung von Kohlendioxid über die Leitung 4b genutzt werden, die ein Gerät mit gasförmigem Kohlendioxid aus dem Erhitzer 5 versorgen soll.

Im letzten Fall führt die Inbetriebnahme von Erhitzer 5 nach einer bestimmten Zeit zum Abtauen, dessen Dauer kontrollierbar sein muß, um zur Versorgung des Anwendungsgeräts mit gasförmigem Kohlendioxid über optimale Anwendungsbedingungen zu verfügen.

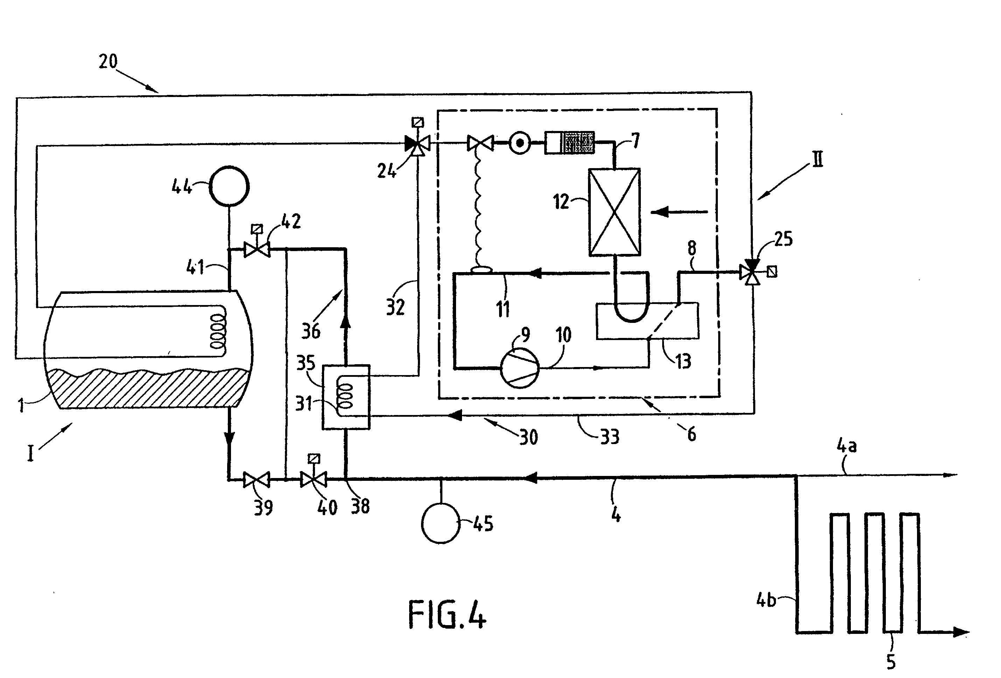

Um dieser Anforderung rechtzeitig gerecht zu werden, wird die Anlage in die in Figur 4 dargestellte Betriebsart geschaltet, in der die Maschine 6 so gesteuert wird, daß sie ständig, jedoch entgegengesetzt zur obigen Situation, an den Kreislauf 30 angeschaltet ist. Zu diesem Zweck wird der Block 13 so gesteuert, daß der Stutzen 10 mit der Leitung 8 und der Stutzen 11 mit der Leitung 7 verbunden ist.

Außerdem werden die Ventile 40 und 42 geöffnet, um die Schleife 36 mit der Abzweigung 41 zu verbinden.

In einer derartigen Situation hat der Betrieb von Maschine 6 zur Folge, daß der Austauscher 31 als Kondensator und der Austauscher 12 als Verdampfer funktioniert, während ein Teil des flüssigen Kohlendioxids über die Schleife 36 fließt und wieder von Behälter 1 aufgenommen wird. Auf seinem Weg durch die Kammer 35 wird dieser Teil des flüssigen Kohlendioxids verdampft und erhöht den Druck der Gasphase an der Decke 3 von Behälter 1. Diese Betriebsphase kann über den Drucksensor 44 gesteuert werden.

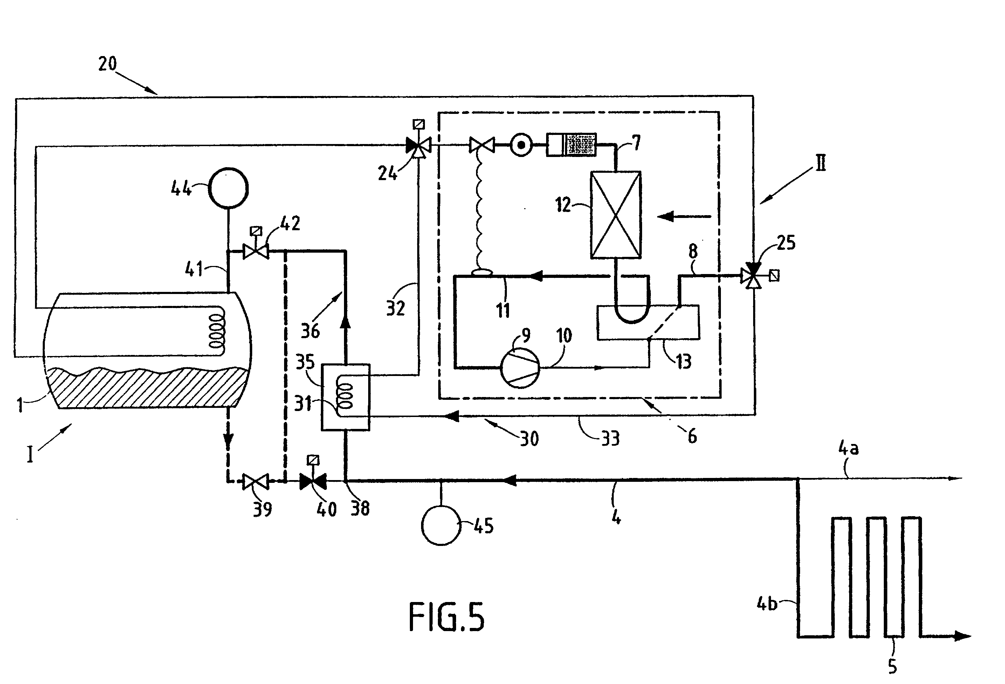

Muß der Lufterhitzer 5 abgetaut werden, beginnt der Abtauzyklus, wie in Figur 5 dargestellt, mit dem Schließen von Ventil 40. In dieser Situation ist die Leitung 4 über die Schleife 36 und die Abzweigung 41 direkt mit der Decke 3 von Behälter 1 verbunden, die an diese Leitung Kohlendioxid in der Gasphase abgibt, das das Abtauen des Erhitzers 5 bewirkt. In diesem Zustand wird die Leitung 4 nur mit der Gasphase versorgt, obwohl der Hahn 39 geöffnet ist, da der Durchsatz der flüssigen Phase durch den Austritt der Gasphase über die Abzweigung 51 aufgrund der vertikalen Richtung der Zweigleitung der Schleife 36 zwischen Hahn 39 und Ventil 40 unterbrochen ist.

Bei einer solchen Betriebsart ist die Maschine 6, wie oben in Verbindung mit Figur 4 gesagt, weiter in Betrieb.

Es kann geschehen, daß bei einer solchen Betriebsart bei der Entnahme durch das Anwendungsgerät ein niedriger Durchsatz aus der Decke 3 mitgenommen wird. In diesem Fall müßte das Regelungssystem von Maschine 6 seinen Betrieb aufgrund einer negativen Austauschbilanz innerhalb des Austauschers 31 unterbrechen. Um eine derartige unerwartete Abschaltung zu vermeiden, wird über das Signal des Temperatursensors 45 die teilweise Öffnung von Ventil 40 gesteuert, so daß über die Schleife 36 und die Anschlußstelle 37, entsprechend der gestrichelten Darstellung, ein Teil flüssiges Kohlendioxid durchgelassen wird, das sich mit der Gasphase vermischt und durch die Kammer 35 strömt.

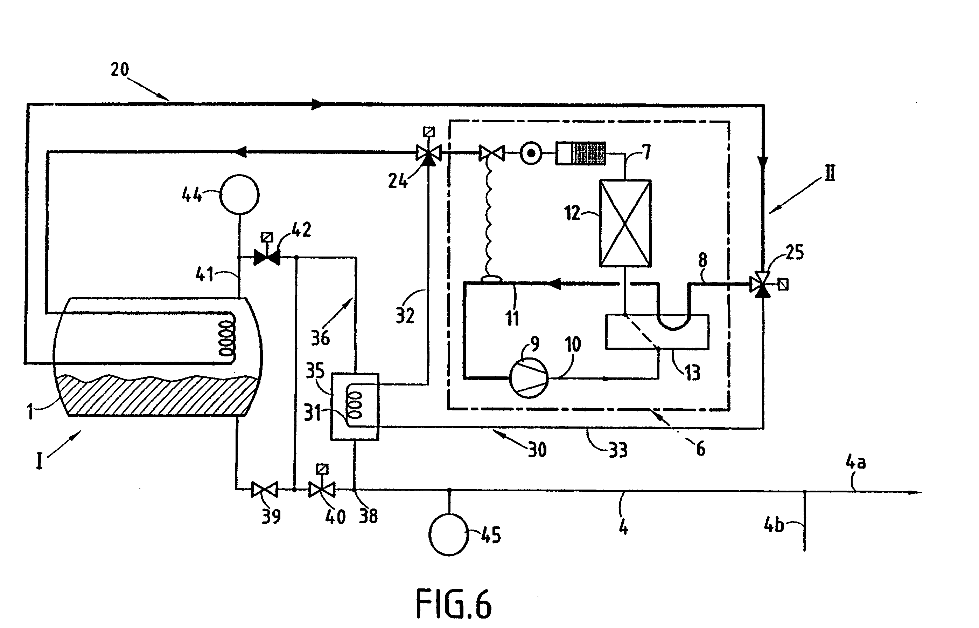

Schließlich ist ein Zustand zu berücksichtigen, in dem sich die Einrichtung außer Betrieb befindet und kein Kohlendioxid entnommen wird. Je nach den Umgebungsbedingungen kann in der Decke 3 von Behälter 1 ein übermäßiger Druckanstieg erfolgen. Zur Regelung dieses Drucks, der vom Sensor 44 erfaßt werden kann, wird die erfindungsgemäße Anlage, wie in Figur 6 dargestellt, in Betrieb genommen. Die Maschine 6 wird durch die Ventile 24 und 25 gesteuert und mit der Leitung 20 verbunden, während durch die Betätigung von Block 13 Leitung 8 mit dem Stutzen 11 und Leitung 7 mit dem Stutzen 10 verbunden wird.

In einem solchen Fall strömt das durch den Verdichter 9 komprimierte Kältemittel durch den Austauscher 12, der als Kondensator wirkt und dieses Kältemittel durch die Leitung 22 zum Austauscher 21 befördert, der sich dann wie ein Verdampfer verhält und die Gasphase an der Decke 3 abkühlt und den Druck innerhalb von Behälter 2 verringert und auf einem optimalen Wert hält.

Das aus dem Verdampfer 21 austretende Kältemittel gelangt über die Leitung 23 und das Ventil 25 in die zweite Leitung 8 und von dort aus über die Leitung 15 zum Einlaßstutzen des Verdichters 9.

## Patentansprüche

1. Anlage zur Regelung des Betriebs einer Einrichtung für die Lagerung und Verteilung von Kohlendioxid, mit einem Lagerbehälter (1) und einer vom Behälter abgehenden Entnahmeleitung (4), **dadurch gekennzeichnet, daß** sie umfaßt:
- eine reversible Kältemaschine (6),
- einen ersten Austauschkreislauf (20) mit einem Austauscher (21) im Teil (3) des Behälters, der von der Gasphase des Kohlendioxids eingenommen wird,
- einen zweiten Austauschkreislauf (30) mit einem Austauscher (31) in der Entnahmeleitung (4) und
- zwei Steuerventile (24, 25) mit einander zugeordneten entgegengesetzten Steuerungen, die zwischen der Kältemaschine und dem ersten bzw. zweiten Kreislauf angeordnet sind.

2. Anlage nach Anspruch 1, **dadurch gekennzeichnet, daß** die reversible Kältemaschine (6) folgendes umfaßt:
- eine erste Strömungsleitung (7) mit einem Austauscher (12) und eine zweite Strömungsleitung (8),
- einen Verdichter (9) und
- einen Umkehrblock (13), der die Verbindung gewährleistet:
• entweder zwischen dem Ausgang (10) des Verdichters und der zweiten Leitung (8) und gleichzeitig zwischen dem Eingang (11) des Verdichters und dem Ausgang des Austauschers (12) der ersten Leitung (7),
• oder zwischen dem Ausgang (10) des Verdichters und dem Eingang des Austauschers (12) der ersten Leitung (7) und gleichzeitig zwischen dem Eingang (11) des Verdichters und der zweiten Leitung (8).

3. Anlage nach Anspruch 2, **dadurch gekennzeichnet, daß** die erste und die zweite Leitung (7 und 8) der Kältemaschine (6) über zwei Dreiwegeventile (24 und 25) jeweils an zwei Austauschkreisläufe (20 bzw. 30) angeschlossen sind.

4. Anlage nach einem der Ansprüche 1 bis 3, **dadurch gekennzeichnet, daß** der zweite Austauschkreislauf (30) einen Austauscher (31) hat, der in einer Kammer (35) in einer Umgehungsschleife (36) in der Entnahmeleitung (4) angeordnet ist, die zwischen den beiden Anschlüssen (37 und 38) der Schleife in der Leitung (4) ein Abschalt-Regelungsventil (40) hat.

5. Anlage nach Anspruch 4, **dadurch gekennzeichnet, daß** die Umgehungsschleife (36) an die Decke (3) des Behälters über eine Abzweigung (41) mit einem Absperrhahn (42) angeschlossen ist.

6. Anlage nach Anspruch 4 oder 5, **dadurch gekennzeichnet, daß** die Umgehungsschleife (36) in der Entnahmeleitung (4) hinter einem Absperrhahn (39) angeordnet ist.

7. Anlage nach Anspruch 1, **dadurch gekennzeichnet, daß** sie einen Temperatursensor (45) hat, der Mittel (43) zur Regelung des Betriebs steuert und in der Entnahmeleitung (4) angeordnet ist.

8. Anlage nach einem der Ansprüche 5 bis 7, **dadurch gekennzeichnet, daß** sie einen Drucksensor (44) hat, der in der Abzweigung (41) angeordnet ist und Mittel (43) zur Regelung des Betriebs steuert.

## Claims

1. Plant for regulating the operation of a facility for the storage and distribution of carbon dioxide, with a storage tank (1) and with a draw-off line (4) emanating from the tank, **characterized in that** it comprises:
- a reversible refrigerating machine (6),
- a first exchange circuit (20) with an exchanger (21) **in that** part (3) of the tank which is occupied by the gas phase of the carbon dioxide,
- a second exchange circuit (30) with an exchanger (31) in the draw-off line (4), and
- two control valves (24, 25) with mutually opposed controls assigned to one another, which are arranged between the refrigerating machine and the first and the second circuit respectively.

2. Plant according to Claim 1, **characterized in that** the reversible refrigerating machine (6) comprises the following:
- a first flow line (7) with an exchanger (12) and a second flow line (8),
- a compressor (9), and
- a reversal block (13) which ensures the connection:
• either between the outlet (10) of the compressor and the second line (8) and at the same time between the inlet (11) of the compressor and the outlet of the exchanger (12) of the first line (7),
• or between the outlet (10) of the compressor and the inlet of the exchanger (12) of the first line (7) and at the same time between the inlet (11) of the compressor and the second line (8).

3. Plant according to Claim 2, **characterized in that** the first and the second line (7 and 8) of the refrigerating machine (6) are connected in each case to two exchange circuits (20 and 30) via two three-way valves (24 and 25).

4. Plant according to one of Claims 1 to 3, **characterized in that** the second exchange circuit (30) has an exchanger (31) arranged in a chamber (35) in a bypass loop (36) in the draw-off line (4) which has a cut-off regulating valve (40) between the two connections (37 and 38) of the loop in the line (4).

5. Plant according to Claim 4, **characterized in that** the bypass loop (36) is connected to the head (3) of the vessel via a branch (41) having a shut-off cock (42).

6. Plant according to Claim 4 or 5, **characterized in that** the bypass loop (36) is arranged downstream of a shut-off cock (39) in the draw-off line (4).

7. Plant according to Claim 1, **characterized in that** it has a temperature sensor (45) which controls operation-regulating means (43) and which is arranged in the draw-off line (4).

8. Plant according to one of Claims 5 to 7, **characterized in that** it has a pressure sensor (44) which is arranged in the branch (41) and which controls operation-regulating means (43).

## Revendications

1. Installation de régulation du fonctionnement d'une installation de stockage et de distribution de dioxyde de carbone comprenant un réservoir de stockage (1) et une conduite de prélèvement (4) partant du réservoir,
**caractérisée par**
- une machine frigorifique réversible (6),
- un premier circuit d'échange (20) comprenant un échangeur (21) dans la partie (3) du réservoir dans laquelle on prélève la phase gazeuse du dioxyde de carbone,
- un second circuit d'échange (30) avec un échangeur (31) dans la conduite de prélèvement (4) et
- deux vannes de commande (24, 25) avec des commandes opposées entre la machine frigorifique et le premier ou le second circuit.

2. Installation selon la revendication 1,
**caractérisée en ce que**
la machine frigorifique réversible (6) comprend :
- une première conduite de passage (7) avec un échangeur (12) et une seconde conduite de passage (8),
- un compresseur (9) et
- un bloc d'inversion (13) qui réalise la liaison :
* soit entre la sortie (10) du compresseur et la seconde conduite (8) et en même temps entre l'entrée (11) du compresseur et la sortie de l'échangeur (12) de la première conduite (7),
* ou entre la sortie (10) du compresseur et l'entrée de l'échangeur (12) de la première conduite (7) et en même temps entre l'entrée (11) du compresseur et la seconde conduite (8).

3. Installation selon la revendication 2,
**caractérisée en ce que**
la première et la seconde conduite (7, 8) de la machine frigorifique (6) sont reliées par deux vannes à trois voies (24, 25) chaque fois à deux circuits d'échange (20, 30).

4. Installation selon l'une quelconque des revendications 1 à 3,
**caractérisée en ce que**
le second circuit d'échange (30) comprend un échangeur (31) placé dans une chambre (35) dans une boucle de contournement (36) de la conduite de prélèvement (4) ayant une vanne de régulation et de coupure (40) entre les deux branchements (37, 38) de la boucle dans la conduite de prélèvement(4).

5. Installation selon la revendication 4,
**caractérisée en ce que**
la boucle de contournement (36) est reliée à la partie supérieure (3) du réservoir par une dérivation (41) munie d'un robinet d'arrêt (42).

6. Installation selon la revendication 4 ou 5,
**caractérisée en ce que**
la boucle de contournement (36) est placée dans la conduite de prélèvement (4) derrière un robinet d'arrêt (39).

7. Installation selon la revendication 1,
**caractérisée en ce qu'**
elle comporte un capteur de température (45) commandant des moyens (43) pour réguler le fonctionnement et qui est prévu dans la conduite de prélèvement (4).

8. Installation selon l'une quelconque des revendications 5 à 7,
**caractérisée en ce qu'**
elle comporte un capteur de pression (44) installé dans la dérivation (41) et commandant des moyens (43) pour réguler le fonctionnement.
